(19) Europäisches Patentamt
European Patent Office
Office européen des brevets

(11) **EP 3 950 122 A1**

(12) **EUROPEAN PATENT APPLICATION**
published in accordance with Art. 153(4) EPC

(43) Date of publication:
**09.02.2022 Bulletin 2022/06**

(21) Application number: **20785298.9**

(22) Date of filing: **26.03.2020**

(51) International Patent Classification (IPC):
**B01J 23/887** (2006.01)  **C01G 53/00** (2006.01)
**C07B 61/00** (2006.01)  **C07C 45/35** (2006.01)
**C07C 47/22** (2006.01)  **C07C 51/25** (2006.01)
**C07C 57/05** (2006.01)

(52) Cooperative Patent Classification (CPC):
**B01J 23/887; C01G 53/00; C07B 61/00;
C07C 45/35; C07C 47/22; C07C 51/25**

(86) International application number:
**PCT/JP2020/013531**

(87) International publication number:
**WO 2020/203606 (08.10.2020 Gazette 2020/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2019  JP 2019065495**

(71) Applicant: **Nippon Kayaku Kabushiki Kaisha
Tokyo 100-0005 (JP)**

(72) Inventors:
• **HIRAOKA, Ryota**
  **Sanyoonoda-shi, Yamaguchi 757-8686 (JP)**
• **FUKUNAGA, Seiichiro**
  **Sanyoonoda-shi, Yamaguchi 757-8686 (JP)**

(74) Representative: **Gille Hrabal
Partnerschaftsgesellschaft mbB
Patentanwälte
Brucknerstraße 20
40593 Düsseldorf (DE)**

(54) **DRIED GRANULES FOR CATALYST PRODUCTION, CATALYST, AND COMPOUND PRODUCTION METHOD**

(57)     Provided is a catalyst in which in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when the SEM image is binarized to be a black-and-white image, a bismuth (Bi) concentration in a white region is larger than a value obtained by adding a value of 2.5 times a standard deviation $\sigma 1$ of the Bi concentration in a black region to the Bi concentration in the black region.

**FIG. 1**

## Description

TECHNICAL FIELD

The present invention relates to a novel catalyst that is highly active and allows for obtaining a desired product in a high yield. In particular, the present invention relates to a catalyst that allows for performing stable production in a high yield even in a region having high catalytic activity, for oxidatively producing an unsaturated aldehyde, an unsaturated carboxylic acid, or a conjugated diene, and a method for producing the same.

BACKGROUND ART

A method for using propylene, isobutylene, t-butyl alcohol or the like as a raw material to produce a corresponding unsaturated aldehyde or unsaturated carboxylic acid, or a catalytic gas phase oxidation for producing 1,3-butadiene from butenes are widely carried out industrially. In particular, regarding the method for using propylene, isobutylene, t-butyl alcohol or the like as a raw material to produce a corresponding unsaturated aldehyde or unsaturated carboxylic acid, many reports have been made for means for improving the yield (for example, Patent Literatures 1, 2, etc.).
Patent Literature 3 discloses that when two or more types of catalysts of the composite metal oxide having different compositions from each other are prepared and the catalyst are filled such that two or more layers are stacked in a tube axis direction for multilayer filling, filling the catalysts such that the amount of a bismuth component with respect to molybdenum decreases from a gas inlet side toward a gas outlet side and the amount of an iron component with respect to molybdenum increases from the gas inlet side toward the gas outlet side keeps the temperature of the reaction bath to be low even in a reaction under high load conditions. In the conventional art, there is no study about a method in which, in the step of using alkene to produce the corresponding unsaturated aldehyde and/or unsaturated carboxylic acid, a total yield of the unsaturated aldehyde and/or unsaturated carboxylic acid (hereinafter referred to as "effective yield") itself is improved and the unsaturated aldehyde and/or unsaturated carboxylic acid is stably produced with the temperature of the reaction bath kept low and with the improved effective yield maintained even in the reaction under high load conditions.

CITATION LIST

PATENT LITERATURE

Patent Literature 1: WO 2016/136882
Patent Literature 2: JP-A-2017-024009
Patent Literature 3: WO 2015/008815

SUMMARY OF INVENTION

TECHNICAL PROBLEM

Even though the improvement is tried by the means as described above, further improvement in yield is required for subjecting alkenes such as propylene and isobutylene and t-butyl alcohol to a partial oxidation to produce the corresponding unsaturated aldehyde and/or unsaturated carboxylic acid and is required in the production of a conjugated diene by oxidative dehydrogenation of butenes. For example, the yield of a target product influences an amount of alkenes such as propylene and isobutylene, t-butyl alcohol, or butenes required for the production and greatly influences the production cost. In addition, continuing the operation for the production at a low yield causes to generate a large amount of by-products to apply a large load to a refining step, and thus this causes a concern that the time and the operating cost required for the refining step increase. Further, some types of by-products may be deposited on the surface of the catalyst or in a gas flow path near the catalyst. The by-products reduce the activity of the catalyst by covering necessary reaction active sites on the surface of the catalyst, and thus this results in the need for forcibly increasing the activity and raising the reaction bath temperature. Then, the catalyst is subjected to thermal stress, and this causes a decrease in life and a further decrease in selectivity, resulting in a decrease in yield. Further, the by-products deposited in the system may also cause an increase in internal pressure, and this may lead to a decrease in selectivity and a decrease in yield. In some cases, a sudden increase in internal pressure may lead to an abnormality in the temperature and the reaction may run out of control. In this case, it is assumed that the operation is stopped for a long period of time and it is necessary to clean the inside of the system and replace the catalyst.

Therefore, an object of the present invention is to provide a catalyst that allows for producing an unsaturated carboxylic acid and/or a conjugated diene stably and inexpensively and allows for obtaining target products in a high yield.

SOLUTION TO PROBLEM

The present inventors have found that the yield of target products is higher in a catalyst in which an uneven distribution of a bismuth (Bi) component can be observed in the evaluation by SEM of catalyst particles than the catalyst in the related art. Thus, the present invention has been completed.

That is, the present invention relates to the following 1) to 11).

1) A catalyst, wherein

in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when the SEM image is binarized to be a black-and-white image, a bismuth (Bi) concentration in a white region is larger than a value obtained by adding a value of 2.5 times a standard deviation $\sigma1$ of the Bi concentration in a black region to the Bi concentration in the black region.

2) A catalyst, wherein

in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when any 10 straight lines are set on the catalyst in the SEM image, a standard deviation $\sigma2$ of the Bi (bismuth) concentration on the straight lines on a catalyst surface is 0.4 or more.

3) A dry granule for catalyst production, wherein

in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when the SEM image is binarized to be a black-and-white image, a bismuth (Bi) concentration in a white region is larger than a value obtained by adding a value of 2.5 times a standard deviation $\sigma1$ of the Bi concentration in a black region to the Bi concentration in the black region.

4) A dry granule for catalyst production, wherein

in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when any 10 straight lines are set on the dry granule in the SEM image, a standard deviation $\sigma2$ of the Bi (bismuth) concentration on the straight lines on a dry granule surface is 0.4 or more.

5) A catalyst produced from the dry granule for catalyst production according to 3) or 4).

6) The catalyst according to any one of 1), 2), and 5), comprising: a catalytically active component, wherein a composition of the catalytically active component is represented by the following formula (1):

$$Mo_{a1}Bi_{b1}Ni_{c1}Co_{d1}Fe_{e1}X_{f1}Y_{g1}Z_{h1}O_{i1} \qquad (1)$$

(in the formula, Mo, Bi, Ni, Co and Fe represent molybdenum, bismuth, nickel, cobalt and iron, respectively; X is at least one element selected from tungsten, antimony, tin, zinc, chromium, manganese, magnesium, silica, aluminum, cerium and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, hi, and i1 represent the number of atoms of molybdenum, bismuth, nickel, cobalt, iron, X, Y, Z, and oxygen, respectively; when $a1 = 12$, $0 < b1 \leq 7$, $0 \leq c1 \leq 10$, $0 < d1 \leq 10$, $0 < c1+d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element).

7) The catalyst according to 6), wherein in the above formula (1), c1, b1 and d1 satisfy the following formula (2):

$$0.300 \leq c1/(b1+d1) \leq 0.600 \qquad (2).$$

8) The catalyst according to any one of 1), 2), and 5) to 7), which is a catalyst in which the catalytically active component is carried on an inert carrier.

9) The catalyst according to any one of 1), 2), and 5) to 8), which is a catalyst for producing an unsaturated aldehyde compound and/or an unsaturated carboxylic acid compound.

10) A method for producing an unsaturated aldehyde compound and/or an unsaturated carboxylic acid compound, wherein the catalyst according to any one of 1), 2), and 5) to 9) is used.

11) The method according to 10), wherein the unsaturated aldehyde compound is acrolein and the unsaturated carboxylic acid compound is acrylic acid.

ADVANTAGEOUS EFFECTS OF INVENTION

The catalyst of the present invention relates to a catalyst for oxidatively producing an unsaturated aldehyde, an unsaturated carboxylic acid, and/or a conjugated diene, and particularly a catalyst that allows for safe and inexpensive production of an unsaturated aldehyde, an unsaturated carboxylic acid and/or a conjugated diene and achieves a high yield of target products.

BRIEF DESCRIPTION OF DRAWINGS

FIG. 1 is an example of an SEM image of the surface of a dry granule for catalyst production.
FIG. 2 is an example of an SEM image of the surface of the dry granule for catalyst production in FIG. 1 in which a hue has been adjusted.
FIG. 3 is an example of an SEM image of the surface of a dry granule for producing a catalyst A.
FIG. 4 is an example of an SEM image of the surface of the dry granule for catalyst production obtained by binarizing FIG. 3 into a black-and-white image.
FIG. 5 is an example of an SEM image of the surface of a dry granule for catalyst producing a catalyst B.
FIG. 6 is an example of an SEM image of the surface of the dry granule for catalyst production obtained by binarizing FIG. 5 into a black-and-white image.
FIG. 7 is an example of an SEM image of the surface of a dry granule for catalyst producing a catalyst C.
FIG. 8 is an example of an SEM image of the surface of the dry granule for catalyst production obtained by binarizing FIG. 7 into a black-and-white image.
FIG. 9 is an example of an SEM image of the surface of a dry granule for catalyst producing a catalyst E.
FIG. 10 is an example of an SEM image of the surface of the dry granule for catalyst production obtained by binarizing FIG. 9 into a black-and-white image.

DESCRIPTION OF EMBODIMENTS

[Uneven Distribution of Bi (bismuth)]

One of the characteristics of a dry granule for catalyst production and a catalyst of the present invention is that bismuth (Bi) is unevenly distributed. Although the detailed reason is unknown, the presence of the uneven distribution portion of bismuth (Bi) allows an oxidation reaction and an oxidative dehydration reaction to proceed efficiently. In the present invention, bismuth (Bi) includes bismuth itself, all compounds and/or oxides of bismuth and other elements contained in the raw material other than bismuth, such as bismuth oxide, bismuth molybdate, and iron bismuth molybdate. The crystal phase thereof is not limited. Hereinafter, bismuth (Bi) may be simply expressed as bismuth or Bi.

In the present invention, as a means for observing the uneven distribution, a method by image analysis using a scanning electron microscope (SEM) can be exemplified, the details of the means are not limited as long as the same accuracy is guaranteed and the means is for the same purpose. For example, an electron probe microanalyzer (EPMA) can be exemplified as another method, and a method using SEM and EPMA in combination can also exemplified. Further, it is presumed that the uneven distribution of bismuth observed in the dry granule for catalyst production is also similarly observed in the catalyst produced using the dry granule. This is because if the observed particles have a certain particle size or less, a step such as application of extremely strong crushing is required to crush these particles, and the dry granule for catalyst production is a composite metal oxide and the particles do not melt unless an excessive amount of heat is applied.

[Method (1) for Measuring Uneven Distribution of Bi]

An SEM image of the dry granule for catalyst production or the catalyst is taken using a scanning electron microscope (SEM) under conditions of an acceleration voltage of 15 kV and a magnification of 1500 times. On the occasion of the taking of image, a cross section can be photographed by embedding the dry granule for catalyst production or the catalyst in a resin or the like as necessary and then sputtering the surface. When there are few uneven distribution portions, it is preferable to photograph the cross section. Further, in order to make the image clearer, gold, platinum or the like may be vapor-deposited. In the present invention, a main component constituting the dry granule for catalyst production and the catalyst is molybdenum, and thus when a backscattered electron image of the SEM image is photographed, Bi, which has a large atomic number, is emphasized in white. In addition, a non-unevenly distributed portion is represented by gray, and a portion where the component having a small atomic number is unevenly distributed or a portion shaded is represented by black. That is, binarizing the obtained SEM image to be a black-and-white image allows for further emphasizing the portion where Bi, which has a large atomic number, is unevenly distributed and allows for more accurately identifying the uneven distribution portion. When the obtained SEM image is binarized, it is more preferable to adjust the contrast and brightness in order to emphasize the light and darkness.

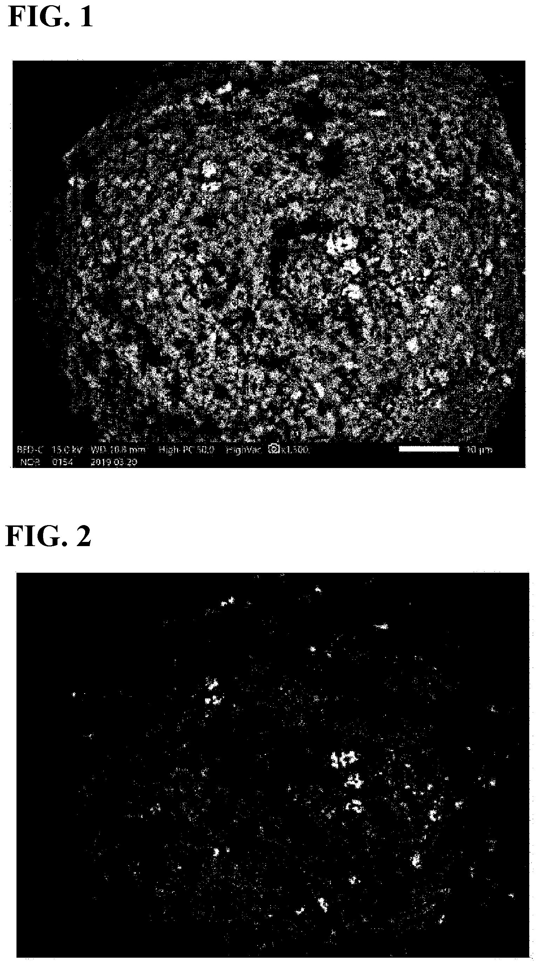

A method of adjusting the SEM image is not specified as long as the uneven distribution portion of Bi can be identified. As an example, an SEM image of the dry granule for catalyst production is attached as FIG. 1, and an SEM image in which the hue of the dry granule for catalyst production is adjusted is attached as FIG. 2 in the description of the present application. In a method of adjusting the hue in the attached FIG. 2, the image of FIG. 2 is obtained by adjusting intermediate tone, contrast and brightness of the SEM image (FIG. 1) to -100, -100 and -50 respectively by Picture manager, developed by Microsoft Corporation. In another method, a SEM image can be binarized to be a black-and-white image by opening the SEM image using Photos developed by Microsoft Corporation, editing the image by adjusting the light to -80 three times repeatedly, reopening the edited image with Paint developed by Microsoft Corporation and saving the edited image as a monotone image (monochrome bitmap).

The Bi concentration of the white region in the image obtained by the above method is measured by EDS (Energy Dispersive X-ray Spectroscopy). The measurement conditions are not limited, but it is preferable to select a spectrum of a metal component used as a raw material of a catalyst except for an alkaline component from the obtained spectrum and determine the relative element ratio. Further, the range to be measured may be appropriately set based on the size of the white region. For example, the measurement point may be set as a point, or the range to be measured may be set within the size of the white region. When the measurement is performed locally, the Bi concentration in the white region is defined as the average value of values measured at least 10 points or more. Particles having a white region as a measurement portion is preferably appropriately determined. The particles having the average particle size of 0.1 $\mu$m or less are difficult to be determined by SEM, and the particles having the average particle size of 1000 $\mu$m or more are likely not to be a component of the catalyst but an impurity or the like.

On the other hand, for comparison with the uneven distribution portion, measurement is performed on the black region in FIG. 2, that is, portion other than the white region in FIG. 2. For the measurement condition, an area equal to or larger than a certain level is needed to be set as the range to be measured such that the uneven distribution portion is not pinpointed and measured. The lower limit of the preferred range of the area to be measured is not limited unless it is a range that is impossible for a device, but should be equal to or larger than the area of the imaged portion of the unevenly distributed particles. Therefore, the area to be measured is preferably 0.01 $\mu$m$^2$ or more. It is preferable to perform EDS measurement on the entire screen to obtain the averaged element proportion in the image, or it is preferable to measure a certain range to be measured of the black region, for example, the size of the white region or more, in order to minimize the influence of the inclusion of the white region. That is, the lower limit of the preferred range of the area to be measured is not limited unless it is a range that is impossible for a device, but should be equal to or larger than the area of the imaged portion of the unevenly distributed particles. Thus, the area to be measured is preferably 0.01 $\mu$m$^2$ or more. The average and wide range of Bi concentration in which the locally uneven distribution of catalyst components and measurement variation of EDS measurement are reduced can be measured by using the Bi concentration measured for the entire screen and the Bi concentration measured for 10 or more black regions as data. In addition, in order to set the range to be measured as described above, it is necessary to measure a certain amount or more of catalyst granule particles in the field of view.

The presence or absence of the uneven distribution is determined by comparing the uneven distribution portion with the other portion by using the component proportion measured in such a manner. That is, it can be expressed that the state in which the Bi concentration in the white region is larger than a value obtained by adding a value of 2.5 times a standard deviation $\sigma 1$ of an average concentration of the Bi concentration in the black region to the average concentration of the Bi concentration in the black region means that the uneven distribution is present. It can be statistically determined that a portion having a concentration outside the range of the average value of $\pm 2.5\sigma 1$ is composed of different component proportion. As described above, a main component constituting the dry granule for catalyst production and the catalyst is molybdenum in the present invention. Thus, when Bi, which has a large atomic number, is binarized into black and white, it is considered that Bi may be abundantly present in the white region and the Bi concentration in the white region is outside of the upper limit. That is, it can be considered that the Bi concentration in the white region is a value larger than the value obtained by adding the value of 2.5 times the standard deviation $\sigma 1$ of the average concentration of the Bi concentration in the black region to the average concentration of the Bi concentration in the black region.

The particle size of the dry granule for catalyst production of the present invention is preferably about 0.1 $\mu$m to 500 $\mu$m, and in this case, the dry granule can be directly measured using SEM.

On the other hand, the catalyst of the present invention is produced by subjecting the above dry granule for catalyst production to a molding step. The catalyst of the present invention has a catalyst particle size of 2 mm to 10 mm, which is relatively large, and thus the catalyst itself is sometimes difficult to be measured using SEM. Thus, a catalyst whose surface has been scraped is needed to be measured. The measuring method is not limited as long as the catalyst is observed by SEM, and a sample in which the surface of the catalyst is scraped by sputtering after being embedded in a resin or the like may be used.

In the present description, "to" represents or more and or less. That is, it means to include the numerical values before and after the "to".

[Method (2) for Measuring Uneven Distribution of Bi]

Line analysis can also be used as a method for measuring the uneven distribution of Bi.

The line analysis is a method in which after obtaining an SEM image in the same manner as in the method (1) for measuring the uneven distribution of Bi, the Bi concentration is analyzed with EDS for any 10 straight lines on the SEM image. The EDS is the same as above. The number of measured points in each of the 10 straight lines is not limited as long as the number of the measured points is 10 points or more, and is preferably about 32 points. If the number of the measured points is more than 32, the time for the measurement increases accordingly. In addition, Pixel Time is preferably 200 ms and the measurement at each point is preferably performed three times. The measurement for 10 lines in each of the vertical, lateral and diagonal directions in each SEM image allows for obtaining stable data can in one image and thus this is preferable.

Based on the analysis value obtained from the integrated value for the line analysis in each direction, the average Bi concentration in each direction and the standard deviation $\sigma 2$ of the average Bi concentration in each direction are obtained. Then, from the data on each of these straight lines, the average Bi concentration and the standard deviation $\sigma 2$ of the average Bi concentration are obtained.

In the present invention, $\sigma 2$ is preferably 0.8 or more. Further, $\sigma 2$ is more preferably 1.5 or more, particularly preferably 2.3 or more, and most preferably 2.5 or more.

[Dry Granule for Catalyst Production]

The dry granule for catalyst production of the present invention is obtained by granulating a slurry obtained by mixing raw materials containing each element constituting the catalyst, using, for example, drum drying, spray drying, evaporation drying, or the like. Bi particles and catalyst particles having a certain size or larger are necessary for uneven distribution of Bi, which is an element constituting the present invention, and thus particles having a certain particle size or more or a slurry having a certain concentration or more are needed to be prepared for achieving to obtain Bi particles and catalyst particles having a certain size or larger. A drying method in regard to the dry granule having a certain particle size or more is appropriately selected. As the drying method, spray drying, in which the slurry can be dried into a granule within a short period of time, is particularly preferable. The form of spray used for spray drying is not limited, but it is preferable to use a disc-shaped spray nozzle.

The temperature for the drying is not limited as long as water can be removed, but may be normal temperature (25°C) in the case of adjusting the pressure and time. However, for removing water more reliably and in a short period of time, 80°C or higher is preferred, and 90°C or higher is more preferred. When the pressure is not adjusted, the temperature is preferably 100°C or higher, and more preferably 150°C or higher.

As will be described in detail in the description of the production process, the dry granule for catalyst production may be further subjected to preliminary calcination, and in this case, the calcination is performed at a temperature of about 150°C to 600°C for about 1 to 12 hours. The SEM measurement for the uneven distribution of Bi is preferably performed after the preliminary calcination, for the catalyst produced in a method having a preliminary calcination step. This is because the preliminarily calcined granule constitutes the catalyst. Hereinafter, the preliminarily calcined granule may be referred to as a preliminarily calcined powder.

[Composition of Catalytically Active Component]

The catalyst of the present invention preferably contains a catalytically active component having a composition represented by the following formula (1).

$$Mo_{a1}Bi_{b1}Ni_{c1}Co_{d1}Fe_{e1}X_{f1}Y_{g1}Z_{h1}O_{i1} \qquad (1)$$

(In the formula, Mo, Bi, Ni, Co and Fe represent molybdenum, bismuth, nickel, cobalt and iron, respectively; X is at least one element selected from tungsten, antimony, tin, zinc, chromium, manganese, magnesium, silica, aluminum, cerium and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, h1, and i1 represent the number of atoms of molybdenum, bismuth, nickel, cobalt, iron, X, Y, Z, and oxygen, respectively; when a1 = 12, $0 < b1 \le 7$, $0 \le c1 \le 10$, $0 < d1 \le 10$, $0 < c1+d1 \le 20$, $0 \le e1 \le 5$, $0 \le f1 \le 3$, $0 \le g1 \le 2$, $0 \le h1 \le 5$, and i1 is a value determined by an oxidation state of each element).

In the above formula (1), preferred ranges of b1 to i1 are as follows.

$0.2 < b1 \le 2$, $1 \le c1 \le 5$, $3 \le d1 \le 8$, $5 \le c1+d1 \le 15$, $0.5 \le e1 \le 4$, $0.01 \le g1 \le 1$, $0 \le f1 \le 1.5$, $0.01 \le g1 \le 1$, and $0 \le h1 \le 2$.

More preferred ranges are as follows.

$0.5 < b1 \le 1$, $2 \le c1 \le 4$, $5 \le d1 \le 7$, $7 \le c1+d1 \le 12$, $1 \le e1 \le 3$, $0 \le f1 \le 1$, $0.02 \le g1 \le 0.2$, and $0 \le h1 \le 1$.

Y is preferably contained in two or less types, and one type is particularly preferred. Further, it is a particularly preferred embodiment that f1 and h1 are 0.

Regarding the catalyst composition in the catalytically active component, c1/(b1+d1) of the above formula (1) preferably satisfy the following formula (2).

$$0.300 \le c1/(b1+d1) \le 0.600 \qquad (2)$$

The upper limit of c1/(b1+d1) is more preferably 0.550, still more preferably 0.540, and particularly preferably 0.530. The lower limit of c1/(b1+d1) is more preferably 0.310. That is, in one of the particularly preferred embodiments, c1/(b1+d1) is 0.310 or more and 0.540 or less.

[Shape and Particle Size of Catalyst]

Examples of the catalyst of the present invention include one obtained by molding the dry granule for catalyst production, one obtained by carrying the dry granule for catalyst production on an inert carrier, one obtained by molding the preliminarily calcined powder, which is made by subjecting the dry granule for catalyst production to preliminary calcination, and one obtained by carrying the preliminarily calcined powder on an inert carrier.

The shape of the catalyst is not limited, but examples thereof include a spherical shape, a columnar shape, and a ring shape. The shape is preferably a spherical shape.

As for the particle size, the average particle size is preferably 1 mm to 15 mm. As for the average particle size, the details do not matter as long as the average particle size is obtained from a measurement of a part of the catalysts sampled at random and, for example, it is sufficient to perform the measurement on 300 or more catalysts. The measuring method is based on the triaxial average size obtained from the average of the length (L), width (B), and thickness (T) of each catalyst sphere. The average particle size is more preferably 2 mm to 10 mm, and particularly preferably 3 mm to 8 mm.

[Carrying]

For the catalyst of the present invention, the above dry granule for catalyst production or the preliminarily calcined powder obtained by preliminary calcination after preparing the catalyst may be used as it is. When the above dry granule for catalyst production or the preliminarily calcined powder is carried on an inert carrier, the carried one is particularly effective as the catalyst of the present invention.

As the material of the inert carrier, known materials such as alumina, silica, titania, zirconia, niobia, silica alumina, silicon carbide, carbides, and mixtures thereof can be used. Further, the particle size, water absorption rate, mechanical strength, crystallinity of each crystal phase, mixing ratio, etc. are not limited, and an appropriate range of these should be selected in consideration of the final catalyst performance, molding properties, production efficiency, etc. The mixing ratio of the carrier and the preliminarily calcined powder is calculated as the carrying ratio according to the following equation based on the charged mass of each raw material.

Carrying ratio (mass%) = (mass of dry granule for catalyst production or preliminarily calcined powder used for the molding)/{(mass of dry granule for catalyst production or preliminarily calcined powder used for the molding) + (mass of carrier used for the molding)] × 100

The preferred upper limit of the carrying ratio is 90%, and the more preferred upper limit is 80%.

The preferred lower limit is 20%, and the more preferred lower limit is 30%.

The inert carrier is preferably silica and/or alumina, and particularly preferably a mixture of silica and alumina.

For the carrying, it is preferred to use a binder. Specific examples of the binder which can be used include water, ethanol, methanol, propanol, a polyhydric alcohol, polyvinyl alcohol as a polymer-based binder, and a silica sol aqueous solution as an inorganic binder; ethanol, methanol, propanol, and a polyhydric alcohol are preferred; a diol such as ethylene glycol and a triol such as glycerin is preferred; and an aqueous solution having a concentration of glycerin of 5 mass% or more is preferred. Using an appropriate amount of a glycerin aqueous solution allows for obtaining a high-performance catalyst having good molding properties and high mechanical strength. The amount of these binders used is usually 2 to 60 parts by mass with respect to 100 parts by mass of the preliminarily calcined powder. The amount of glycerin aqueous solution is preferably 10 to 30 parts by mass.

The binder and the preliminarily calcined powder may be alternately or simultaneously supplied to a molding machine on the occasion of carrying.

[Method for Producing Catalyst]

The starting raw material for each element constituting the dry granule for catalyst production, the preliminarily calcined powder or the catalyst of the present invention is not limited. For example, as a raw material of a molybdenum component, molybdenum oxides such as molybdenum trioxide, molybdic acid or salts thereof such as molybdate, ammonium paramolybdate and ammonium metamolybdate, and heteropolyacids containing molybdenum or salts thereof such as phosphomolybdic acid and silicate molybdic acid, can be used.

As a raw material of a bismuth component, bismuth salts such as bismuth nitrate, bismuth carbonate, bismuth sulfate, bismuth acetate and basic bismuth nitrate, bismuth trioxide, metal bismuth and the like can be used. These raw materials can be used as solids or as an aqueous solution, a nitric acid solution, or a slurry of bismuth compounds generated from those aqueous solutions, and the nitrate, a solution thereof, or a slurry obtained from the solution is preferably used.

As a starting raw material for other constituent elements, ammonium salt, nitrate, nitrite, carbonate, subcarbonate, acetate, chloride, inorganic acid, inorganic acid salt, heteropolyacid, heteropolyacid salt, sulfate, hydroxide, organic acid salt, and oxide of metallic elements commonly used in this type of catalyst may be used, or a mixture thereof may be used in combination. Ammonium salts and nitrates are preferably used.

A compound containing these active components may be used alone or in combination of two or more. A slurry liquid can be obtained by uniformly mixing each compound containing an active component and water. The amount of water to be used in the slurry liquid is not limited as long as the total amount of the compound to be used can be completely dissolved or uniformly mixed. The amount of water to be used may be appropriately determined in consideration of the drying method and drying conditions, and is usually 100 parts by mass or more and 2000 parts by mass or less with respect to 100 parts by mass of the total mass of the compound for preparing the slurry. Too large amount of water causes many disadvantages that not only dry particles having a particle size of a certain size or more cannot be obtained, but also the energy cost of the drying step becomes high, and the particles cannot be sometimes completely dried.

The slurry liquid of the source compound of the above each component element is preferably prepared by (a) a method of mixing each of the above source compounds at once, (b) a method of mixing the above source compounds at once and then performing aging, (c) a method of mixing the above source compounds stepwise, (d) a method of repeating mixing step and aging step stepwise, and (e) a method combining (a) to (d). Here, the above aging means "an operation in which industrial raw materials or semi-finished products are processed under specific conditions such as a certain period of time and a certain temperature to conduct acquisition or improvement of the required physical properties and chemical properties, or proceeding of a predetermined reaction". In the present invention, the above certain period of time means a range of 5 minutes or longer and 24 hours or shorter, and the above certain temperature means a range from room temperature to a point equal to or lower than a boiling point of an aqueous solution or an aqueous dispersion liquid. Among these, in terms of the activity and yield of the finally obtained catalyst, preferred is the (c) method of mixing the above source compounds stepwise, more preferred is a method in which each raw material to be mixed with a mother liquid stepwise is completely dissolved to be a solution, and most preferred is a method of mixing various mixed solutions of alkali metal solution and nitrate with a mother liquid in which the raw material of the molybdenum is a mixed solution or slurry. However, it is not always necessary to mix all the elements constituting the catalyst in this step, and some elements or some amounts thereof may be added in the subsequent steps.

In the present invention, the shape of the stirring blade of the stirrer used in mixing the essential active components is not limited. Any stirring blade such as a propeller blade, a turbine blade, a paddle blade, an inclined paddle blade, a screw blade, an anchor blade, a ribbon blade, a large lattice blade can be used in one stage or in two or more stages of which blades are the same or different types in the vertical direction. In addition, a baffle (obstruction plate) may be installed in the reaction tank if necessary.

Then, the slurry liquid thus obtained is dried. The drying method is not limited so long as the slurry liquid can be completely dried by the method, but examples thereof include drum drying, freeze drying, spray drying and evaporation drying. Among these, spray drying, which allows the slurry liquid to be dried into a powder or granule within a short period of time, is particularly preferred in the present invention. The temperature of the drying with spray drying varies depending on the concentration of the slurry liquid, the liquid sending speed, or the like. Typically, the temperature of the inlet air used for drying is 200°C or higher and 300°C or lower, and the temperature of the air at the outlet of a drying machine is 70°C or higher and 150°C or lower. Further, the method of the slurry liquid atomizer in spray drying is not limited as long as those skilled in the art have known about the method. For example, a disk-type atomizer, a two-fluid atomizer, and an ultrasonic atomizer are preferred, and a disk-type atomizer is most preferred, and the disk rotation speed is 5,000 rpm to 30,000 rpm. In this case, the slurry liquid is preferably dried such that the average particle size of a slurry liquid dried product (catalyst precursor) to be obtained is 1 $\mu$m to 1000 $\mu$m. Optimizing the above slurry preparation conditions and spraying conditions is exemplified as one of the methods for achieving the uneven distribution of Bi in the present invention.

For example, a particularly effective method is optimizing the rotation speed of the atomizer in the case of spray drying. The rotation of the atomizer varies depending on the composition of the catalyst precursor, but is preferably 10,000 rpm or more and 20,000 rpm or less. The more preferred upper limit of the rotation speed of the atomizer is 18,000 pm, the particularly preferred upper limit of the rotation speed of the atomizer is 17,000 rpm, and the most preferred upper limit of the rotation speed of the atomizer is 16,000 rpm. The more preferred lower limit of the rotation speed is 11000 rpm, the particularly preferred lower limit of the rotation speed is 12000 rpm, and the most preferred lower limit of the rotation speed is 13000 rpm. That is, the most preferred range of the rotation speed of the atomizer is 13000 rpm or more and 16000 rpm or less.

Subjecting the catalyst precursor obtained as described above to preliminary calcination, molding, and then main calcination allows for controlling and holding the obtained shape, and obtaining a catalyst having particularly excellent mechanical strength for industrial use, and the catalyst can exhibit stable catalyst performance.

As described above, as for the molding, either a carrying shaping in which the preliminarily calcined powder is carried on a carrier such as silica or a non-carrying shaping in which no carrier is used can be adopted. Specific examples of the molding method include tablet molding, press molding, extrusion molding and granulation molding. As the shape of the molded product, for example, a columnar shape, a ring shape, a spherical shape or the like can be appropriately selected in consideration of operating conditions. Preferred is a carried catalyst in which a catalytically active component is carried on a spherical carrier, particularly an inert carrier such as silica or alumina and in which the average particle size is preferably 1.0 mm or more and 15.0 mm or less, more preferably 2.0 mm or more and 10.0 mm or less, and particularly preferably 3.0 mm or more and 8.0 mm or less. As for the carrying method, a tumbling granulation method, a method using a centrifugal flow coating apparatus, a wash coating method, and the like are widely known. The method is not limited as long as the preliminarily calcined powder can be uniformly carried on the carrier, but the tumbling granulation method is preferred in consideration of the production efficiency of the catalyst and the like. Specifically, the tumbling granulation method is a method in which using a device that has a flat or uneven disk at the bottom of a fixed cylindrical container, a carrier charged into the container is vigorously agitated by means of a repeat of rotation motion and revolution motion of the carrier itself by rotating the disk at a high speed, and then the preliminarily calcined powder is added into the container to carry the powder component on the carrier.

On the occasion of carrying, it is preferred to use a binder. Specific examples of the binder which can be used include water, ethanol, methanol, propanol, a polyhydric alcohol, polyvinyl alcohol as a polymer-based binder and a silica sol aqueous solution as an inorganic binder; ethanol, methanol, propanol, and a polyhydric alcohol are preferred; a diol such as ethylene glycol and a triol such as glycerin is more preferred; and an aqueous solution of glycerin having a concentration of 5 mass% or more is still more preferred. Using an appropriate amount of the glycerin aqueous solution allows for obtaining a high-performance catalyst having good molding properties and high mechanical strength. The amount of these binders to be used is usually 2 to 60 parts by mass with respect to 100 parts by mass of the preliminarily calcined powder, and the amount of the glycerin aqueous solution is preferably 15 to 50 parts by mass. The binder and the preliminarily calcined powder may be alternately supplied to a molding machine or simultaneously supplied to the molding machine on the occasion of the carrying. Further, on the occasion of molding, a small amount of known additives such as graphite and talc may be added. None of a molding aid, a pore-forming agent and a carrier added in the molding is considered as the constituent element of the active component in the present invention, regardless of whether the molding aid, the pore-forming agent and the carrier have the activity in the sense of converting the raw material into some other product.

The preliminary calcination method, the preliminary calcination conditions, the main calcination method, and the main calcination conditions are not limited, but known treatment methods and conditions can be applied. The preliminary calcination or the main calcination is usually carried out at 150°C or higher and 600°C or lower, and preferably 200°C or higher and 550°C or lower, for 0.5 hours or longer, and preferably 1 hour or longer and 40 hours or shorter under the conditions that an oxygen-containing gas such as air or an inert gas flows. Here, the inert gas refers to a gas that does not reduce the reaction activity of the catalyst, and specific examples thereof include nitrogen, carbon dioxide, helium and argon. The optimum conditions for the main calcination vary depending on the reaction conditions when an unsaturated aldehyde and/or an unsaturated carboxylic acid and a conjugated diene are produced using a catalyst, and changing the process parameters of the main calcination step, that is, the oxygen content in the atmosphere, the maximum temperature reached and the calcination time falls within the scope of the present invention, because the changing is well-known for the skilled person. The main calcination step shall be carried out after the above preliminary calcination step, and the maximum temperature reached (main calcination temperature) in the main calcination step shall be higher than the maximum temperature reached (preliminary calcination temperature) in the above preliminary calcination step. The technique of the calcination includes but not limited to a fluidized bed, rotary kiln, muffle furnace, and tunnel firing furnace, and should be selected within an appropriate range in consideration of the final catalyst performance, mechanical strength, molding properties, production efficiency and the like.

The catalyst of the present invention is preferably used as a catalyst for producing an unsaturated aldehyde compound, an unsaturated carboxylic acid compound, and a conjugated diene, is more preferably used in a first stage, that is, as a catalyst for producing an unsaturated aldehyde compound, and is particularly preferably used as a catalyst for producing acrolein from propylene.

[Catalyst in Second Stage]

When the catalyst of the present invention is used as a catalyst for producing an unsaturated aldehyde compound, an unsaturated carboxylic acid compound can be obtained by performing a second-stage oxidation reaction.

In this case, the catalyst of the present invention can be used as the catalyst in the second stage, but a catalyst containing a catalytically active component represented by the following formula (3) is preferable.

$$Mo_{12}V_{a2}W_{b2}Cu_{c2}Sb_{d2}X_{e2}Y_{f2}Z_{g2}O_{h2} \qquad (3)$$

(In the formula, Mo, V, W, Cu, Sb and O represent molybdenum, vanadium, tungsten, copper, antimony and oxygen, respectively; X represents at least one element selected from the group consisting of an alkali metal and thallium; Y represents at least one element selected from the group consisting of magnesium, calcium, strontium, barium and zinc; and Z represents at least one element selected from the group consisting of niobium, cerium, tin, chromium, manganese, iron, cobalt, samarium, germanium, titanium and arsenic. a2, b2, c2, d2, e2, f2, g2 and h2 represent the atomic proportion of each element, and with respect to molybdenum atom 12, a satisfies $0 < a2 \leq 10$, b2 satisfies $0 \leq b2 \leq 10$, c2 satisfies $0 < c2 \leq 6$, d2 satisfies $0 < d2 \leq 10$, e2 satisfies $0 \leq e2 \leq 0.5$, f2 satisfies $0 \leq f2 \leq 1$, and g2 satisfies $0 \leq g2 < 6$. Further, h2 is the number of oxygen atoms required to satisfy the atomic value of each component.)

In the production of a catalyst containing the catalytically active component represented by the above formula (3), a method widely known as a method for preparing this kind of a catalyst, for example, an oxide catalyst or a catalyst having a heteropolyacid or a salt structure thereof, can be adopted. The raw materials that can be used in producing the catalyst are not limited, and various materials can be used. For example, molybdenum oxides such as molybdenum trioxide, molybdic acid or salts thereof such as molybdic acid and an ammonium molybdate, molybdenum-containing heteropolyacids or salts thereof such as phosphomolybdic acid and silicomolybdic acid, and the like can be used. The raw material of an antimony component is not limited, but antimony trioxide or antimony acetate is preferred. As raw materials for other elements such as vanadium, tungsten, copper and the like, nitrate, sulfate, carbonate, phosphate, organic acid salt, halide, hydroxide, oxide or the metal of these elements can be used.

A compound containing these active components may be used alone or in combination of two or more.

Next, the slurry liquid obtained above is dried to obtain a solid of catalytically active component. The drying method is not limited so long as the slurry liquid can be completely dried by the method. However, examples thereof include drum drying, freeze drying, spray drying and evaporation drying. Spray drying is preferred because the slurry liquid can be dried into a powder or granule in a short period of time. The temperature of the drying with spray drying varies depending on the concentration of the slurry liquid, the liquid sending speed or the like, but the temperature at the outlet of a drying machine is approximately 70°C to 150°C. In this case, the slurry liquid is preferably dried such that the average particle size of a slurry liquid dried product (catalyst precursor) to be obtained is 1 $\mu$m to 1000 $\mu$m.

The solid of catalytically active component in the second stage obtained as described above can be used as it is for a coating mixture, and is preferably subjected to calcination because the molding properties may be improved. The calcination method and the calcination conditions are not limited, and known treatment methods and conditions can be applied. The optimum calcination conditions vary depending on the used raw material for the catalyst, catalyst composition, preparation method and the like. The calcination temperature is usually 100°C to 350°C, preferably 150°C to 300°C, and the calcination time is usually 1 to 20 hours. The calcination is usually carried out in an air atmosphere, but may be carried out in an atmosphere of an inert gas such as nitrogen, carbon dioxide, helium or argon. The calcination in an air atmosphere may be carried out after calcination in an inert gas atmosphere, if necessary. The thus-obtained calcined solid is preferably pulverized before the molding. The pulverizing method is not limited, but it is preferable to use a ball mill.

The compound containing the active component in preparing the slurry for the second stage does not necessarily have to contain all the active components, and a part of the components may be used before the following molding step.

The shape of the catalyst in the second stage is not limited. The catalyst is used by being molded into a columnar shape, a tablet, a ring shape, a spherical shape or the like in order to reduce the pressure loss of a reaction gas in the oxidation reaction. Among these, the solid of catalytically active component is particularly preferably carried on an inert carrier to be a carried catalyst because improvement in selectivity and removal of heat of reaction can be expected. A tumbling granulation method described below is preferred for the carrying. This method is a method in which, for example, in a device that has a flat or uneven disk at the bottom of a fixed container, a carrier in the container is vigorously agitated by repeatedly performing rotation motion and revolution motion by rotating the disk at a high speed, and then a mixture for the carrying including the binder, the solid of catalytically active component and optionally a molding aid and/or a strength improver is carried on the carrier. As a method of adding the binder, any methods may be adapted such as 1) premixing a binder with the mixture for the carrying, 2) adding the binder at the same time as the mixture for the carrying is added into the fixed container, 3) adding the binder after adding the mixture for the carrying into the fixed container, 4) adding the binder before adding the mixture for the carrying into the fixed container, 5) dividedly preparing the mixture for the carrying and the binder independently and adding the whole amount of them in the appropriate combination of 2) to 4). Among these, for example, 5) is preferably performed by adjusting the adding rate using an auto feeder or the like such that the mixture for the carrying does not adhere to the wall of the fixed container and the mixture for the carrying does not aggregate with each other and a predetermined amount of the mixture for the carrying is carried on the carrier.

Examples of the binder include water, ethanol, polyhydric alcohol, polyvinyl alcohol as a polymer-based binder, celluloses such as a crystalline cellulose, methyl cellulose and ethyl cellulose, and a silica sol aqueous solution as an inorganic binder. A monohydric alcohol such as ethanol, a dihydric alcohols such as ethylene glycol, a trihydric alcohol such as glycerin and polyhydric alcohols such as cellulose are preferred, and an aqueous solution in which a trihydric or lower alcohol is used at a concentration of 5 wt% or more is particularly preferred. The amount of the binder to be used is usually 2 to 60 parts by mass, and preferably 10 to 50 parts by mass with respect to 100 parts by mass of the mixture for the carrying.

Specific examples of the carrier in the above carrying include a spherical carrier having a diameter of 1 mm to 15 mm, and preferably 2 mm to 10 mm, such as silicon carbide, alumina, silica alumina, mullite and arandom. The carriers having a porosity of 10% to 70% are usually used. The carrier and the mixture for the carrying at the ratio of the mixture for the carrying/(mixture for the carrying + carrier) =10 mass% to 75 mass% are usually used, and the carrier and the mixture for the carrying at the ratio of the mixture for the carrying/(mixture for the carrying + carrier) =15 mass% to 60 mass% are preferably used. When the ratio of the mixture for the carrying tends to be large, the reaction activity of the carried catalyst is large, but the mechanical strength tends to be small. On the contrary, when the ratio of the mixture for the carrying is small, the mechanical strength tends to be large, but the reaction activity tends to be small.

In the above, examples of the molding aid to be used as necessary include silica gel, diatomite, and alumina powder. The amount of the molding aid to be used is usually 1 to 60 parts by mass with respect to 100 parts by mass of the solid of catalytically active component. If necessary, the use of inorganic fibers (for example, ceramic fibers or whiskers) that are inactive to the solid of catalytically active component and the reaction gas as the strength improver is useful for improving the mechanical strength of the catalyst, and glass fibers are preferred. The amount of the fiber to be used is usually 1 to 30 parts by mass with respect to 100 parts by mass of the solid of catalytically active component. None of a molding aid, a pore-forming agent and a carrier added in the molding of the catalyst for the first stage is considered as the constituent element of the active component in the present invention, regardless of whether the molding aid, the pore-forming agent and the carrier have the activity in the sense of converting the raw material into some other product.

The carried catalyst obtained as described above can be used as a catalyst for the catalytic gas phase oxidation, and is preferably subjected to calcination because the molding properties may be improved. The calcination method and the calcination conditions are not limited, and known treatment methods and conditions can be applied. The optimum calcination conditions vary depending on the raw material for the catalyst to be used, the catalyst composition, the preparation method, and the like, but the calcination temperature is usually 100°C to 450°C, preferably 270°C to 420°C, and the calcination time is usually 1 to 20 hours. The calcination is usually carried out in an air atmosphere, and may be carried out in an atmosphere of an inert gas such as nitrogen, carbon dioxide, helium or argon. The calcination in an air atmosphere may be carried out after the calcination in an inert gas atmosphere, if necessary.

When the catalyst of the present invention is used in a reaction of using propylene, isobutylene, t-butyl alcohol, butene, and the like as raw materials to produce the corresponding unsaturated aldehyde, unsaturated carboxylic acid, and conjugated diene, and particularly, in a reaction of producing acrolein and acrylic acid by catalytic gas phase oxidation of propylene with molecular oxygen or a gas containing molecular oxygen, using the catalyst of the present invention allows for improving the catalytic activity and the yield, and is very effective in improving the price competitiveness of the product as compared with the known method. In addition, the effect of improving the process stability of the partial oxidation reaction accompanied by heat generation, such as reduction of the hot spot temperature can be expected. Further, the catalyst of the present invention is also effective in reducing by-products that adversely influences the environment and the quality of the final product, such as carbon monoxide (CO), carbon dioxide ($CO_2$), acetaldehyde, acetic acid, and formaldehyde.

In the method for producing acrolein and/or acrylic acid of the present invention, the method for flowing the raw material gas may be an ordinary single-flow method or a recycling method, and can be carried out under widely used conditions, and is not limited. For example, a mixed gas containing 1 vol% to 10 vol% and preferably 4 vol% to 9 vol% of propylene, 3 vol% to 20 vol% and preferably 4 vol% to 18 vol% of molecular oxygen, 0 vol% to 60 vol% and preferably 4 vol% to 50 vol% of water vapor at room temperature as a starting raw material, and 20 vol% to 80 vol% and preferably 30 vol% to 60 vol% of an inert gas such as carbon dioxide and nitrogen is introduced to the catalyst of the present invention filled in a reaction tube at 250°C to 450°C under normal pressure to 10 atm and a space velocity of 300 to 5000 $h^{-1}$ to perform a reaction.

In the present invention, unless otherwise specified, the improvement of the catalytic activity means that the conversion rate of the raw material is high when the catalytic reaction is carried out at the same temperature of the reaction bath.

In the present invention, unless otherwise specified, a high yield means that the total yield of the corresponding unsaturated aldehyde and/or unsaturated carboxylic acid and conjugated diene is high when the oxidation reaction is performed using propylene, isobutylene, t-butyl alcohol, butene, and the like as raw materials.

In the present invention, unless otherwise specified, the constituent elements of the catalytically active component refer to all the elements to be used in the method for producing a catalyst, but the raw materials and the constituent elements thereof that disappear, sublimate, volatilize, and burn at the maximum temperature or lower in the main calcination step are not included in the constituent elements of the catalytically active component. Further, silicon and the other elements constituting inorganic materials contained in the molding aid and the carrier in the shaping step are not included in the constituent elements of the catalytically active component.

In the present invention, the hot spot temperature refers to the maximum temperature in the temperature distribution in the catalyst-filled bed that is measured in thermocouples installed in the multi-tube reaction tube in the long axis direction, and the temperature of the reaction bath refers to a set temperature of a heat medium used for the purpose of cooling the heat generated in the reaction tube. The number of measuring points in the temperature distribution is not limited, but for example, the catalyst filling length is evenly divided from 10 to 1000.

In the present invention, the unsaturated aldehyde and the unsaturated aldehyde compound refers to organic compounds having at least one double bond and at least one aldehyde in the molecule, such as acrolein and methacrolein. In the present invention, the unsaturated carboxylic acid and the unsaturated carboxylic acid compound refers to organic compounds having at least one double bond and at least one carboxy group or an ester group of the carboxyl group in the molecule, and are, for example, acrylic acid, methacrylic acid, and methyl methacrylate.

Examples

Hereinafter, Examples will be shown with specific examples, but the present invention is not limited to Examples without departing from the spirit thereof.

[Example of Catalyst Production 1]

800 parts by weight of ammonium heptamolybdate tetrahydrate was completely dissolved in 3040 parts by weight of pure water warmed to 60°C. Subsequently, 5.59 parts by weight of potassium nitrate was dissolved in 55 ml of pure water and added to the above solution. Subsequently, 274.6 parts by weight of ferric nitrate nonahydrate, 571.5 parts by weight of cobalt nitrate hexahydrate, and 307.4 parts by weight of nickel nitrate hexahydrate were dissolved in 611 ml of pure water warmed to 60°C. These solutions were gradually mixed while being stirred. Subsequently, a solution prepared by completely dissolving 311.4 parts by weight of bismuth nitrate in a nitric acid aqueous solution that was prepared by adding 79.3 parts by weight of nitric acid (60 wt%) to 330 ml of pure water was added to the above solution and mixed with stirring. This slurry was dried by disk type spray drying, and the obtained dry powder was subjected to preliminary calcination at a maximum temperature 440°C for 4 hours. Five wt% of a crystalline cellulose with respect to the preliminarily calcined powder was added to the preliminarily calcined powder, followed by thoroughly being mixed. The mixture was carried and molded into a spherical shape on an inert spherical carrier of 4.5 mm by using a 30 wt% glycerin solution as a binder by a tumbling granulation method so that a carrying ratio was 50 wt% . Subsequently, the resultant one was calcined at a maximum temperature of 530°C for 4 hours to obtain a spherical catalyst A having an average particle size of 5.2 mm. The catalytically active component calculated from the charged raw materials was a composite metal oxide having the following atomic proportion.
Mo:Bi:Fe:Co:Ni:K = 12:1.7:2.0:5.2:2.8:0.15
The drying conditions by spray drying were as follows.

Rotation speed of atomizer: 14000 rpm
Supply amount of raw material: 14 kg/hr

[Example of Catalyst Production 2]

800 parts by weight of ammonium heptamolybdate tetrahydrate was completely dissolved in 3040 parts by weight of pure water warmed to 60°C. Subsequently, 11.0 parts by weight of cesium nitrate was dissolved in 100 ml of pure water and added to the above solution. Subsequently, 259.3 parts by weight of ferric nitrate nonahydrate, 791.3 parts by weight of cobalt nitrate hexahydrate, and 109.8 parts by weight of nickel nitrate hexahydrate were dissolved in 615 ml of pure water warmed to 60°C. These solutions were gradually mixed while being stirred. Subsequently, a solution prepared by completely dissolving 311.4 parts by weight of bismuth nitrate in a nitric acid aqueous solution that was prepared by adding 79.3 parts by weight of nitric acid (60 wt%) to 330 ml of pure water was added to the above solution and mixed with stirring. This slurry was dried by disk type spray drying, and the obtained dry powder was subjected to preliminary calcination at a maximum temperature 440°C for 4 hours. Five wt% of a crystalline cellulose with respect to the preliminarily calcined powder was added to the preliminarily calcined powder, followed by thoroughly being mixed. The mixture was carried and molded into a spherical form on an inert spherical carrier of 4.0 mm by using a 30 wt% glycerin solution as a binder by a tumbling granulation method so that a carrying ratio was 40 wt%. Subsequently, the resultant one was calcined at a maximum temperature of 520°C for 4 hours to obtain a spherical catalyst B having an average particle size of 4.4 mm. The catalytically active component calculated from the charged raw materials was a composite metal oxide having the following atomic proportion.
Mo:Bi:Fe:Co:Ni:Cs = 12:1.7:1.7:7.2:1.0:0.15
The drying conditions by spray drying were as follows.

Rotation speed of atomizer: 14000 rpm
Supply amount of raw material: 14 kg/hr

[Example of Catalyst Production 3]

800 parts by weight of ammonium heptamolybdate tetrahydrate was completely dissolved in 3040 parts by weight of pure water warmed to 60°C. Subsequently, 11.0 parts by weight of cesium nitrate was dissolved in 100 ml of pure water and added to the above solution. Subsequently, 259.3 parts by weight of ferric nitrate nonahydrate, 769.3 parts by weight of cobalt nitrate hexahydrate, and 109.8 parts by weight of nickel nitrate hexahydrate were dissolved in 615 ml of pure water warmed to 60°C. These solutions were gradually mixed while being stirred. Subsequently, a solution prepared by completely dissolving 274.8 parts by weight of bismuth nitrate in a nitric acid aqueous solution that was prepared by adding 70.0 parts by weight of nitric acid (60 wt%) to 300 ml of pure water was added to the above solution and mixed with stirring. This slurry was dried by disk type spray drying, and the obtained dry powder was subjected to preliminary calcination at a maximum temperature 440°C for 4 hours. Five wt% of a crystalline cellulose with respect to the preliminarily calcined powder was added to the preliminarily calcined powder, followed by thoroughly being mixed. The mixture was carried and molded into a spherical form on an inert spherical carrier of 3.8 mm by using a 30 wt% glycerin solution as a binder by a tumbling granulation method so that a carrying ratio was 50 wt% . Subsequently, the resultant one was calcined at a maximum temperature of 520°C for 4 hours to obtain a spherical catalyst C having an average particle size of 4.5 mm. The catalytically active component calculated from the charged raw materials was a composite metal oxide having the following atomic proportion.
Mo:Bi:Fe:Co:Ni:Cs = 12:1.5:1.7:7.0:1.0:0.15

The drying conditions by spray drying were as follows.

Rotation speed of atomizer: 11000 rpm
Supply amount of raw material: 14 kg/hr

[Example of Catalyst Production 4]

800 parts by weight of ammonium heptamolybdate tetrahydrate was completely dissolved in 3040 parts by weight of pure water warmed to 60°C. Subsequently, 3.68 parts by weight of cesium nitrate was dissolved in 33 ml of pure water and added to the above solution. Subsequently, 305.1 parts by weight of ferric nitrate nonahydrate, 714.4 parts by weight of cobalt nitrate hexahydrate, and 274.5 parts by weight of nickel nitrate hexahydrate were dissolved in 686 ml of pure water warmed to 60°C. These solutions were gradually mixed while being stirred. Subsequently, a solution prepared by completely dissolving 183.2 parts by weight of bismuth nitrate to a nitric acid aqueous solution that was prepared by adding 46.6 parts by weight of nitric acid (60 wt%) to 194 ml of pure water was added to the above solution and mixed with stirring. This slurry was dried by disk type spray drying, and the obtained dry powder was subjected to preliminary calcination at a maximum temperature 440°C for 4 hours. Five wt% of a crystalline cellulose with respect to the preliminarily calcined powder was added to the preliminarily calcined powder, followed by thoroughly being mixed. The mixture was carried and molded into a spherical form on an inert spherical carrier of 3.8 mm by using a 30 wt% glycerin solution as a binder by a tumbling granulation method so that a carrying ratio is 50 wt% . Subsequently, the resultant one was calcined at a maximum temperature of 540°C for 4 hours to obtain a spherical catalyst D having an average particle size of 4.5 mm. The catalytically active component calculated from the charged raw materials was a composite metal oxide having the following atomic ratio.
Mo:Bi:Fe:Co:Ni:Cs = 12:1.0:2.0:6.5:2.5:0.05

The drying conditions by spray drying were as follows.

Rotation speed of atomizer: 11000 rpm
Supply amount of raw material: 14 kg/hr

[Example of Catalyst Production 5]

A catalyst E was prepared in the same manner as the catalyst A, except that spray drying was carried out by using a two-fluid nozzle.
The drying conditions by the two-fluid nozzle were as follows.

Supply amount of raw material: 5.0 kg/h
Supply amount of atomizing air: 46 $Nm^3$/h
Pressure: 0.3 MPa

[Example 1]

(Oxidation Reaction Test of Propylene)

A stainless steel reactor having an inner diameter of 28.4 mm provided with a jacket for circulating an alumina powder by air as a heat medium and a thermocouple for measuring the temperature of the catalyst layer on the tube axis, was filled with 35 ml of the catalyst A and the temperature of the reaction bath was set to 320°C. A gas in which an amount of propylene, air and water to be supplied was set such that the molar proportion of the raw materials satisfied propylene:oxygen:nitrogen:water = 1:2.0:10.8:1.5 was introduced into an oxidation reactor at a space velocity of 2110

h$^{-1}$, the outlet pressure of the reactor was set to 0 kPaG, and the catalyst performance was evaluated 20 hours after the start of the reaction. Thereafter, the temperature of the reaction bath was set to 330°C and the catalyst performance was further evaluated 24 hours after the start of the reaction. When the conversion rate of the raw material was 70%, the effective yield was 67.1% and the calorific value in the reactor (value obtained by subtracting the temperature of the reaction bath from the hot spot temperature) was 82°C (Table 1).

[Comparative Example 1]

The catalyst performance was evaluated in the same manner as in Example 1 except that the molded catalyst E was used. When the conversion rate of the raw material was 70%, the effective yield was 66.7% and the calorific value in the reactor was 88°C (Table 1).

[Example 2]

(Oxidation Reaction Test of Isobutylene)

A stainless steel reactor having an inner diameter of 22 mm provided with a jacket for circulating an alumina powder by air as a heat medium and a thermocouple for measuring the temperature of the catalyst layer on the tube axis, was filled with 35 ml of the molded catalyst B and the temperature of the reaction bath was set to 350°C. A gas in which an amount of isobutylene, air, water and nitrogen to be supplied was set such that the molar proportion of the raw materials satisfied isobutylene:oxygen:nitrogen:water = 1:2.2:12.5:1.0 was introduced into an oxidation reactor at a space velocity of 1200 h$^{-1}$, the inlet pressure of the reactor was set to 0.05 MPa, and the catalyst performance was evaluated 20 hours after the start of the reaction. The conversion rate of the raw material was 99.3%, and the effective yield was 82.7%. The calorific value in the reactor was 61°C (Table 1).

[Example 3]

(Oxidative Dehydrogenation Reaction Test of 1-butene)

A stainless steel reactor having an inner diameter of 28.4 mm provided with a jacket for circulating an alumina powder by air as a heat medium and a thermocouple for measuring the temperature of the catalyst layer on the tube axis, was filled with 53 ml of the molding catalyst C and the temperature of the reaction bath was set to 340°C. A gas in which an amount of 1-butene, air, water and nitrogen to be supplied was set such that the molar proportion of the raw materials satisfied 1-butene:oxygen:nitrogen:water = 1:1:7:1 was introduced into an oxidation reactor at a space velocity of 1440 h$^{-1}$, the outlet pressure of the reactor was set to 0 kPaG, and the catalyst performance was evaluated 15 hours after the start of the reaction. The conversion rate of the raw material was 93.4%, and the effective yield was 81.5%. The calorific value in the reactor was 46°C (Table 1).

[Example 4]

The catalyst performance was evaluated in the same manner as in Example 3, except that the molded catalyst D was used and the temperature of the reaction bath was set to 320°C. The conversion rate of the raw material was 96.1%, and the effective yield was 80.6%. The calorific value in the reactor was 83°C (Table 1).

<Method (1) for Measuring Uneven Distribution of Bi>

The uneven distribution measurement (1) of Bi of each dry granule for catalyst production was performed as follows.

I) The dry granule for producing the corresponding catalyst were observed by SEM at the following magnifications.

catalyst A: 4500 times
catalyst B: 3300 times
catalyst C: 3200 times
catalyst D: 4100 times
catalyst E: 7200 times

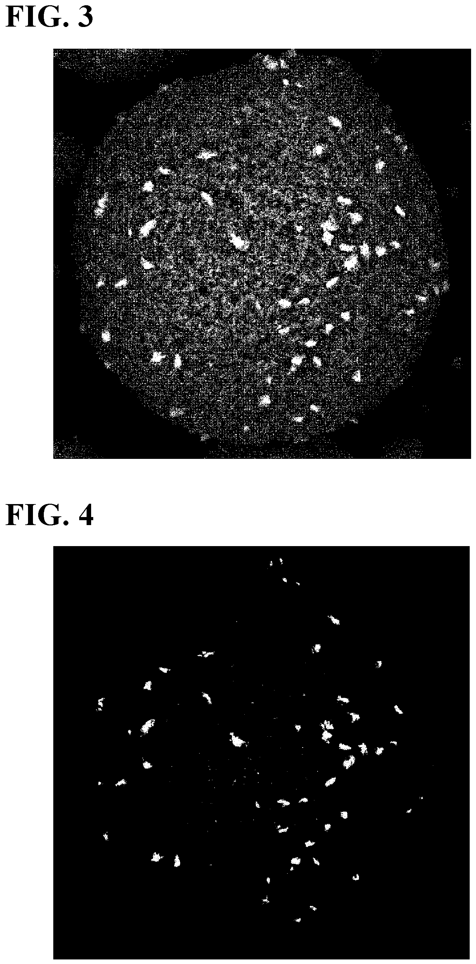

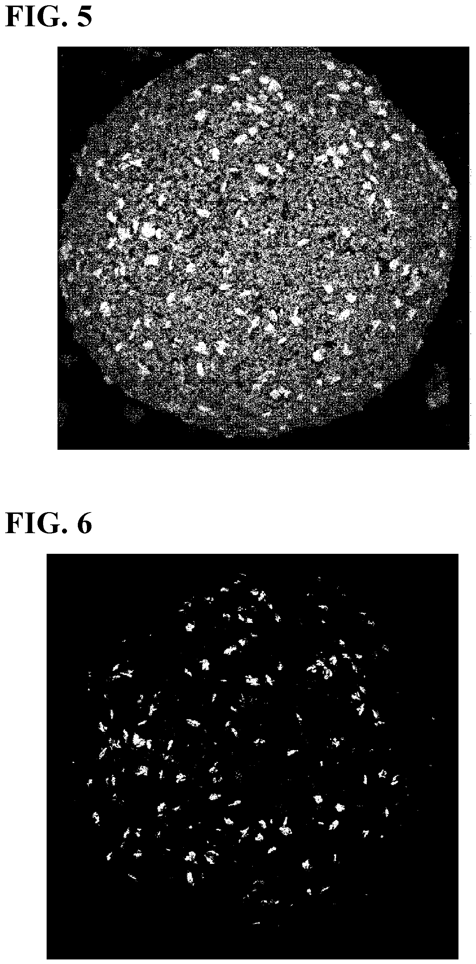

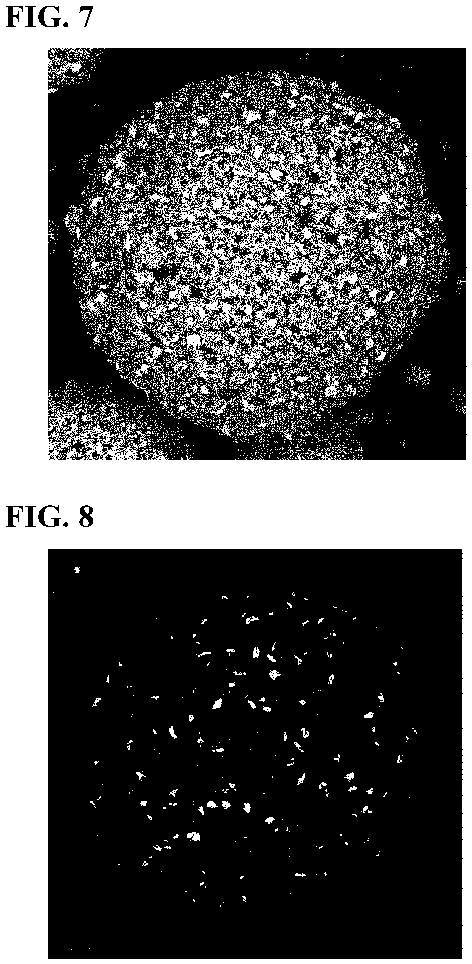

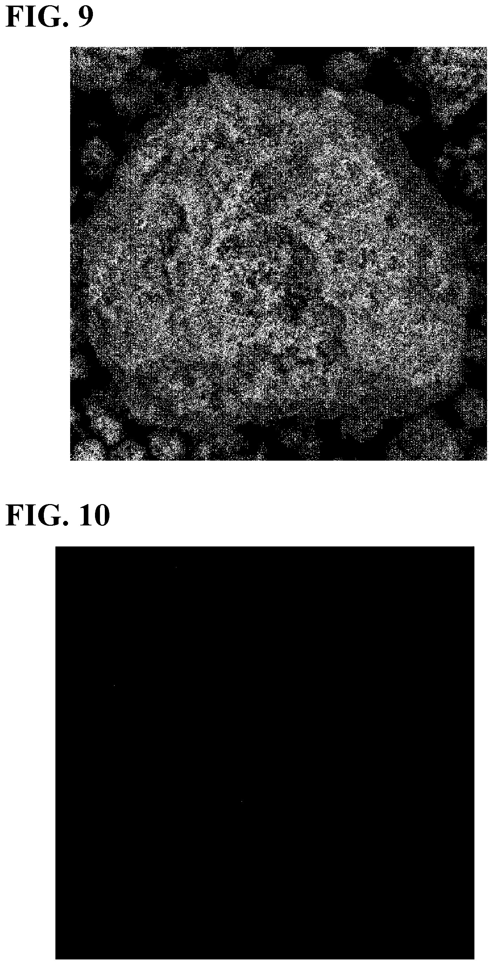

FIG. 3, FIG. 5, FIG. 7 and FIG. 9 were SEM images corresponding to the dry granules for producing the catalyst A, the catalyst B, the catalyst C, and the catalyst E, respectively.

II) The SEM image saved in the bitmap format was opened with Photo developed by Microsoft Corporation, and the image was edited by repeatedly setting the light to -80 three times. The image obtained by this operation was binarized to a black-and-white image by reopening the image with Paint manufactured by Microsoft Corporation and saving the image as a monotone image (monochrome bitmap). FIG. 4, FIG. 6, FIG. 8 and FIG. 10 are binarized SEM images corresponding to the dry granules for producing the catalyst A, the catalyst B, the catalyst C and the catalyst E, respectively.

III) EDS measurements were performed at 10 points on the surface of the dry granules corresponding to the white region and the black region of the binarized image, and the average Bi concentration (%) was obtained. Table 1 shows the average Bi concentration in the white region and the black region. In addition, the standard deviation $\sigma 1$ of the Bi concentration in the black region was determined. The results are shown in Table 1.

<Method (2) for Measuring Uneven Distribution of Bi>

I) The same SEM images as those in the method (1) above were used.

II) Lines were drawn vertically, horizontally and diagonally on the SEM image of the dry granules, and EDS measurement was performed on a plurality of points on the line under the following conditions, and the average of the Bi concentration (%) of each point was obtained. The results are shown in Table 1. In addition, the standard deviation $\sigma 2$ of the Bi concentration at each point was determined. The results are shown in Table 1.

Pixel Time: 200 ms
Number of data points per line: 32 points
Number of repeated measurements at each point: 3 times

[Table 1]

| | | | Example 1 | Comparative Example 1 | Example 2 | Example 3 | Example 4 |
|---|---|---|---|---|---|---|---|
| | Raw material | | Propylene | Propylene | Isobutylene | 1-butene | 1-butene |
| | Conversion rate of the raw material (%) | | 70.0 | 70.0 | 99.3 | 93.4 | 96.1 |
| | Effective yield (%) | | 67.1 | 66.7 | 82.7 | 81.5 | 80.6 |
| | Calorific value (°C) | | 82 | 88 | 61 | 46 | 83 |
| Dry granule for catalyst production | Uneven distribution measurement (1) of Bi | Bi concentration (%) in white region | 34.08 | 8.73 | 14.93 | 24.6 | 6.49 |
| | | Bi concentration (%) in black region | 4.84 | 7.55 | 7.00 | 5.00 | 3.55 |
| | | Bi concentration $\sigma 1$ in black region | 0.83 | 0.55 | 0.89 | 1.87 | 0.32 |
| | Uneven distribution measurement (2) of Bi | Bi concentration (%) in line analysis | 12.6 | 12.9 | 10.7 | 10.3 | 7.2 |
| | | Bi concentration $\sigma 2$ in line analysis | 1.25 | 0.31 | 1.39 | 1.55 | 0.76 |

EP 3 950 122 A1

From the results in Table 1, it was observed in Example 1 in which the Bi concentration in the white region measured by EDS was larger than a value (4.84 + 2.08) obtained by adding a value of 2.5 times the standard deviation $\sigma 1$ of the Bi concentration in the black region to the Bi concentration in the black region, in acrolein production by the oxidation reaction of propylene, that the yield was high and the calorific value was low. On the other hand, it was observed in Comparative Example 1 in which the Bi concentration in the white region was lower than a value (7.55 + 1.38) obtained by adding a value of 2.5 times the standard deviation $\sigma 1$ of the Bi concentration in the black region to the Bi concentration in the black region, that the yield is poor and the calorific value is high.

In addition, the standard deviation $\sigma 2$ of the Bi concentration obtained by line analysis was 0.4 or more in Example 1, whereas the standard deviation $\sigma 2$ of the Bi concentration obtained by line analysis was less than 0.4 in Comparative Example 1.

Similarly, in the production of methacrolein by the oxidation reaction of isobutylene (Example 2) and in the production of butadiene by the oxidative dehydrogenation reaction of 1-butene (Examples 3 and 4), a high yield and a low calorific value were observed in the catalyst in which the uneven distribution of Bi was observed. The calorific value is a difference between the maximum temperature in the reaction tube and the temperature of the reaction bath observed by the thermocouple.

Although the present invention has been described in detail with reference to specific examples, it is apparent to those skilled in the art that it is possible to add various alterations and modifications without departing from the spirit and the scope of the present invention.

The present application is based on Japanese Patent Application (No. 2019-065495) filed on March 29, 2019, the entire contents of which are incorporated herein by reference. In addition, all references cited here are entirely incorporated.

INDUSTRIAL APPLICABILITY

When the catalyst of the present invention is used to oxidatively produce an unsaturated aldehyde compound, an unsaturated carboxylic acid compound, or a conjugated diene compound, the product can be obtained in a high yield. Further, the hot spot temperature (the heat generating part in the catalyst layer) is low, and thus the life of the catalyst is extended, so that the target product can be stably produced for a long period of time.

**Claims**

1. A catalyst, wherein

   in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when the SEM image is binarized to be a black-and-white image, a bismuth (Bi) concentration in a white region is larger than a value obtained by adding a value of 2.5 times a standard deviation $\sigma 1$ of the Bi concentration in a black region to the Bi concentration in the black region.

2. A catalyst, wherein

   in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when any 10 straight lines are set on the catalyst in the SEM image, a standard deviation $\sigma 2$ of the Bi (bismuth) concentration on the straight lines on a catalyst surface is 0.4 or more.

3. A dry granule for catalyst production, wherein

   in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when the SEM image is binarized to be a black-and-white image, a bismuth (Bi) concentration in a white region is larger than a value obtained by adding a value of 2.5 times a standard deviation $\sigma 1$ of the Bi concentration in a black region to the Bi concentration in the black region.

4. A dry granule for catalyst production, wherein

in a scanning electron microscope (SEM) image of the catalyst, the image being obtained by using an SEM in which an acceleration voltage is set to 15 kV under a magnification at which a maximum vertical length of one particle in the SEM image occupies 90% or more of a vertical length of the SEM image, and a maximum lateral length of one particle in the SEM image occupies 90% or more of a lateral length of the SEM image, when any 10 straight lines are set on the dry granule in the SEM image, a standard deviation $\sigma 2$ of the Bi (bismuth) concentration on the straight lines on a dry granule surface is 0.4 or more.

5. A catalyst produced from the dry granule for catalyst production according to claim 3 or 4.

6. The catalyst according to any one of claims 1, 2, and 5, comprising: a catalytically active component, wherein a composition of the catalytically active component is represented by the following formula (1):

$$Mo_{a1}Bi_{b1}Ni_{c1}Co_{d1}Fe_{e1}X_{f1}Y_{g1}Z_{h1}O_{i1} \qquad (1)$$

(in the formula, Mo, Bi, Ni, Co and Fe represent molybdenum, bismuth, nickel, cobalt and iron, respectively; X is at least one element selected from tungsten, antimony, tin, zinc, chromium, manganese, magnesium, silica, aluminum, cerium and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, hi, and i1 represent the number of atoms of molybdenum, bismuth, nickel, cobalt, iron, X, Y, Z, and oxygen, respectively; when a1 = 12, $0 < b1 \leq 7$, $0 \leq c1 \leq 10$, $0 < d1 \leq 10$, $0 < c1+d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element).

7. The catalyst according to claim 6, wherein in the above formula (1), c1, b1 and d1 satisfy the following formula (2):

$$0.300 \leq c1/(b1+d1) \leq 0.600 \qquad (2).$$

8. The catalyst according to any one of claims 1, 2, and 5 to 7, which is a catalyst in which the catalytically active component is carried on an inert carrier.

9. The catalyst according to any one of claims 1, 2, and 5 to 8, which is a catalyst for producing an unsaturated aldehyde compound and/or an unsaturated carboxylic acid compound.

10. A method for producing an unsaturated aldehyde compound and/or an unsaturated carboxylic acid compound, wherein the catalyst according to any one of claims 1, 2, and 5 to 9 is used.

11. The method according to claim 10, wherein the unsaturated aldehyde compound is acrolein and the unsaturated carboxylic acid compound is acrylic acid.

# FIG. 1

# FIG. 2

# FIG. 3

# FIG. 4

## FIG. 5

## FIG. 6

# FIG. 7

# FIG. 8

**FIG. 9**

**FIG. 10**

**INTERNATIONAL SEARCH REPORT**

| International application No. |
| --- |
| PCT/JP2020/013531 |

A. CLASSIFICATION OF SUBJECT MATTER
Int.Cl. B01J23/887(2006.01)i, C01G53/00(2006.01)i, C07B61/00(2006.01)i,
C07C45/35(2006.01)i, C07C47/22(2006.01)i, C07C51/25(2006.01)i,
C07C57/05(2006.01)i
FI: B01J23/887Z, C01G53/00A, C07C45/35, C07C47/22A, C07C51/25, C07C57/05,
C07B61/00300
According to International Patent Classification (IPC) or to both national classification and IPC

B. FIELDS SEARCHED

Minimum documentation searched (classification system followed by classification symbols)
Int.Cl. B01J23/887, C01G53/00, C07B61/00, C07C45/35, C07C47/22, C07C51/25,
C07C57/05

Documentation searched other than minimum documentation to the extent that such documents are included in the fields searched
Published examined utility model applications of Japan     1922–1996
Published unexamined utility model applications of Japan   1971–2020
Registered utility model specifications of Japan           1996–2020
Published registered utility model applications of Japan   1994–2020

Electronic data base consulted during the international search (name of data base and, where practicable, search terms used)


C. DOCUMENTS CONSIDERED TO BE RELEVANT

| Category* | Citation of document, with indication, where appropriate, of the relevant passages | Relevant to claim No. |
| --- | --- | --- |
| X | WO 2017/010159 A1 (NIPPON KAYAKU CO., LTD.) 19.01.2017 (2017-01-19), claims, paragraphs [0007]-[0018] | 1–11 |
| X | CN 103894205 A (CHINA PETROLEUM & CHEMICAL) 02.07.2014 (2014-07-02), claims, paragraphs [0008]-[0031] | 1–11 |
| A | WO 2016/136882 A1 (NIPPON KAYAKU CO., LTD.) 01.09.2016 (2016-09-01), entire text | 1–11 |
| A | WO 03/070369 A1 (MITSUBISHI RAYON CO., LTD.) 28.08.2003 (2003-08-28), entire text | 1–11 |
| A | WO 2014/181839 A1 (NIPPON KAYAKU CO., LTD.) 13.11.2014 (2014-11-13), entire text | 1–11 |

☒ Further documents are listed in the continuation of Box C.    ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08.06.2020 | 16.06.2020 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2020/013531 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2015/008814 A1 (NIPPON KAYAKU CO., LTD.) 22.01.2015 (2015-01-22), entire text | 1-11 |
| A | JP 2015-147188 A (NIPPON KAYAKU CO., LTD.) 20.08.2015 (2015-08-20), entire text | 1-11 |
| A | CN 104437581 A (CHINA PETROLEUM & CHEMICAL) 25.03.2015 (2015-03-25), entire text | 1-11 |

Form PCT/ISA/210 (second sheet) (January 2015)

| INTERNATIONAL SEARCH REPORT | International application No. |
|---|---|
| Information on patent family members | PCT/JP2020/013531 |

```
WO 2017/010159 A1  19.01.2017    US 2018/0186712 A1
                                 claims, paragraphs [0038]-[0055]
                                 EP 3321247 A1
                                 CN 107848920 A
                                 KR 10-2018-0029031 A

CN 103894205 A     02.07.2014    (Family: none)

WO 2016/136882 A1  01.09.2016    US 2018/0029018 A1
                                 the whole sentence
                                 EP 3263213 A1
                                 KR 10-2017-0125827 A
                                 CN 107405609 A

WO 03/070369 A1    28.08.2003    US 2005/0159619 A1
                                 the whole sentence
                                 KR 10-2004-0082438 A
                                 CN 1633336 A
                                 JP 4515769 B2

WO 2014/181839 A1  13.11.2014    US 2016/0059218 A1
                                 the whole sentence
                                 EP 2995375 A1
                                 KR 10-2015-0131119 A
                                 CN 105209168 A

WO 2015/008814 A1  22.01.2015    US 2016/0145180 A1
                                 the whole sentence
                                 EP 3023405 A1
                                 KR 10-2016-0014666 A
                                 CN 105392761 A

JP 2015-147188 A   20.08.2015    (Family: none)

CN 104437581 A     25.03.2015    (Family: none)
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016136882 A
- JP 2017024009 A
- WO 2015008815 A
- JP 2019065495 A